(19) Europäisches Patentamt / European Patent Office / Office européen des brevets

(11) **EP 3 938 206 B1**

(12) **EUROPÄISCHE PATENTSCHRIFT**

(45) Veröffentlichungstag und Bekanntmachung des Hinweises auf die Patenterteilung:
**10.05.2023   Patentblatt 2023/19**

(21) Anmeldenummer: **20707665.4**

(22) Anmeldetag: **06.03.2020**

(51) Internationale Patentklassifikation (IPC):
**B32B 18/00** (2006.01)  **C01C 3/02** (2006.01)
**B01D 69/04** (2006.01)  **B01D 71/02** (2006.01)
**C10G 9/16** (2006.01)  **B01D 67/00** (2006.01)
**B01D 69/02** (2006.01)  **C01B 3/24** (2006.01)
**C01B 3/34** (2006.01)  **C10G 2/00** (2006.01)
**C10G 9/20** (2006.01)  **C10G 9/36** (2006.01)

(52) Gemeinsame Patentklassifikation (CPC):
**B32B 18/00; B01D 67/0041; B01D 69/02;
B01D 69/04; B01D 71/021; B01D 71/024;
B01D 71/025; C01B 3/24; C01B 3/34; C01C 3/02;
C10G 2/32; C10G 9/16; C10G 9/203; C10G 9/36;**
B01D 2325/02;                           (Forts.)

(86) Internationale Anmeldenummer:
**PCT/EP2020/056003**

(87) Internationale Veröffentlichungsnummer:
**WO 2020/187607 (24.09.2020 Gazette 2020/39)**

(54) **GASDICHTES, WÄRMEDURCHLÄSSIGES, KERAMISCHES UND MEHRSCHICHTIGES VERBUNDROHR**

GAS-TIGHT, HEAT-PERMEABLE MULTILAYER CERAMIC COMPOSITE TUBE

TUBE COMPOSITE CÉRAMIQUE ET MULTICOUCHE, ÉTANCHE AUX GAZ ET DIATHERMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2019   EP 19163157**

(43) Veröffentlichungstag der Anmeldung:
**19.01.2022   Patentblatt 2022/03**

(73) Patentinhaber: **BASF SE**
**67056 Ludwigshafen am Rhein (DE)**

(72) Erfinder:
• **KOLIOS, Grigorios**
  **67056 Ludwigshafen (DE)**
• **LAIB, Heinrich**
  **67117 Limburgerhof (DE)**
• **SCHEIFF, Frederik**
  **67056 Ludwigshafen (DE)**
• **ZOELS, Bernd**
  **67056 Ludwigshafen (DE)**
• **KERN, Matthias**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
**WO-A1-2016/184776     WO-A1-2017/007649
JP-A- 2003 053 166**

EP 3 938 206 B1

(52) Gemeinsame Patentklassifikation (CPC): (Forts.)
B01D 2325/04; B01D 2325/06; B01D 2325/22;
C01B 2203/0233; C01B 2203/0238;
C01B 2203/0266; C04B 2235/5224;
C04B 2235/5228; C04B 2235/5244;
C04B 2235/5248; C04B 2235/9607;
C04B 2237/341; C04B 2237/343; C04B 2237/38;
C04B 2237/586; C04B 2237/64; C04B 2237/704;
C04B 2237/765; C04B 2237/84; F16L 9/10;
F16L 9/14; Y02P 20/141

**Beschreibung**

Die vorliegende Erfindung betrifft ein gasdichtes mehrschichtiges Verbundrohr oder Bereiche eines mehrschichtigen Verbundrohres mit einem Wärmedurchgangskoeffizient von > 500 W/m$^2$/K beinhaltend mindestens zwei Schichten, welches in seinem Aufbau über den Querschnitt der Wand des Verbundrohres als innere Schicht eine unporöse monolithische Oxid-Keramik aufweist, welche von einer äußeren Schicht aus oxidischer Faserverbundkeramik umschlossen wird, wobei diese äußere Schicht eine offene Porosität ε (im Sinne von DIN EN 623-2) von größer als 5% und kleiner als 50%, bevorzugt größer als 10% und kleiner als 30% aufweist und die innere Oberfläche des Verbundrohres mehrere zur Außenwand des Verbundrohres gerichtete Vertiefungen aufweist.

Endotherme Reaktionen stehen häufig am Anfang der Wertschöpfungskette der chemischen Industrie, beispielsweise bei der thermischen Spaltung von Ethan, Propan, Butan, Naphtha und schwersiedenderen Erdölfraktionen, der Reformierung von Erdgas, der Dehydrierung von Propan, der Dehydroaromatisierung von Methan zu Benzol oder der Pyrolyse von Kohlenwasserstoffen. Diese Reaktionen sind stark endotherm und laufen bei hohen Temperaturen ab, d.h. Temperaturen zwischen 500° C und 1700° C sind erforderlich, um technisch und wirtschaftlich interessante Ausbeuten zu erreichen.

Zum Beispiel enthält die thermische Spaltung von Kohlenwasserstoffen, dem sogenannten Steamcracking oder Dampfspalten, parallele endotherme Crack- und Dehydrierreaktionen, die bei leichtem Überdruck und hohen Temperaturen durchgeführt werden. Der Standardprozess nach dem Stand der Technik ist das Steamreaforming von Ethan, Propan oder Naphtha (nichthydriertes Rohbenzin) zur Herstellung von Ethylen, Propylen und C4-Olefinen. Diese Produkte zählen zu den mengenmäßig wichtigsten Vorprodukten in der chemischen Industrie. Die Steamcracker gehören zu den Chemieanlagen mit dem größten Stoffdurchsatz. Im Stand der Technik wird dieser stark endotherme Prozess in Rohrschlagen durchgeführt, die von außen durch Feuerung beheizt werden. In einem sogenannten Spaltofen werden mehrere parallel verlaufende Rohre gleichzeitig beheizt und innen von einem Einsatzstoff-Dampf-Gemisch durchströmt. Die Funktion der Rohrwände ist dabei die Übertragung des Wärmestroms von einer externen Wärmequelle in das Reaktionsvolumen und die hermetische Trennung des Reaktionsvolumens von der umgebenden Wärmequelle unter Erhaltung der Druckdifferenz zwischen den zwei Räumen. Die Rohre der Festbettreaktoren sind typischerweise zylindrisch mit variablem oder einheitlichem Durchmesser über die gesamte Rohrlänge. Innerhalb des Ofens können auch verschieden Rohre geteilt oder zusammengeführt werden. Der Werkstoff der Rohre ist typischerweise hochlegierter austenitischer Schleuderguss.

Technische Spaltprozesse werden bei Drücken bis 5 bar Überdruck und Temperaturen bis 1000 ° C, wobei dieser Wert die Produktgastemperatur am Austritt der Reaktionsrohre repräsentiert. Der technische Prozess ist vor allem kinetisch limitiert. Der Begriff "kinetisch limitiert" bedeutet, dass die Verweilzeit des Reaktionsgases in den Spaltrohren so kurz ist, dass die Spalt- und Dehydrierreaktionen nicht das thermodynamische Gleichgewicht erreichen.

Bei der Verwendung von metallischen Reaktorwerkstoffen ist die maximale äußere Rohrwandtemperatur auf ca. 1050-1100 ° C beschränkt.

Allerdings wäre eine höhere maximale äußere Rohrwandtemperatur (engl. Tube metal temperature, abgekürzt TMT) aus mehreren Gründen wünschenswert: insbesondere um die notwendige Wärme für die Spaltreaktionen trotz Koksablagerungen auf der Rohrinnenseite bereitzustellen Zum einen führt die Koksablagerung an der heißen Rohrwand führt dazu, dass das die äußere Rohrwandtemperatur im Laufe des Betriebs angehoben werden muss, um die thermische Isolationswirkung des Kokses zu kompensieren. Das führt zu einer höheren Feuerungsleistung und zu einem höheren Energieverbrauch. Zum zweiten führt die Koksablagerung dazu, dass der Ofen beim Erreichen der maximal zulässigen äußeren Rohrwandtemperatur außer Betrieb genommen und durch Abbrennen mit Luft entkokt werden muss.

Rohrwand-Temperaturen von > 1100° C erfordern den Einsatz keramischer Werkstoffe, vorzugsweise von Oxidkeramiken. Die Vorteile von keramischen Werkstoffen, insbesondere Oxidkeramiken, sind hohe Temperaturbeständigkeit bis 1800 ° C, chemische Passivität, Korrosionsbeständigkeit und hohe Festigkeit. Der größte Nachteil von keramischen Werkstoffen ist deren hohe Sprödigkeit. Diese Eigenschaft wird über die Bruchzähigkeit $K_{IC}$ beschrieben, die beispielsweise gemäß DIN EN ISO 12737 für Metalle, bzw. gemäß DIN EN ISO 15732 für monolithische Keramiken bestimmt wird. Für Stahl, einem Vertreter zäher Werkstoffe, beträgt $K_{IC} \approx 50 \, MPa \, \sqrt{m}$. Für monolithische Keramiken, beispielsweise Zirkonoxid ($ZrO_2$) oder Korund ($Al_2O_3$) beträgt $K_{IC} \cong 3 - 5 \, MPa \, \sqrt{m}$. Hierdurch sind monolithische Keramiken ungeeignet für Druckapparate mit einem Druck von > 0,5 bar, da diese Werkstoffe das Kriterium "Riss vor Bruch" nicht gewährleisten können, sondern ein plötzlicher, sich nicht ankündigender Bruch stattfinden könnte.

Eine Alternative sind Faserverbundkeramiken bestehend aus oxidischen Fasern, die in einer porösen Matrix aus oxidischer Keramik eingebettet sind. Die offene Porosität ε von Faserverbundkeramiken kann in der Regel Werte zwischen 5% und 50% annehmen. Die Vorteile von Faserverbundkeramiken sind hohe Temperaturbeständigkeit bis 1300 ° C oder darüber, hohe Temperaturwechselbeständigkeit und ein quasi-duktiles Verformungs- und Bruchverhalten.

Die Bruchzähigkeit von Faserverbundkeramiken kann Werte erreichen von $K_{IC} \cong 10 - 50\ MPa\ \sqrt{m}$ . Als Folge der porösen Struktur besitzen Faserverbundkeramiken eine geringere Dichte, einen niedrigeren Elastizitätsmodul und einen niedrigeren Wärmeleitfähigkeitskoeffizient gegenüber monolithischen Keramiken mit derselben chemischen Zusammensetzung. Die Tabelle 1 enthält eine Liste der relevanten Normen für die Bestimmung dieser Parameter.

Tabelle 1: Liste der relevanten Normen für die Bestimmung struktureller, mechanischer und thermophysikalischer Parameter für monolithische Keramiken und Verbundkeramiken.

| Parameter | Monolithische Keramik | Faserverbundkeramik |
|---|---|---|
| Dichte, offene Porosität | DIN EN 623-2 | DIN V ENV 1389 |
| E-Modul | DIN V ENV 843-2 | DIN EN 658-1 |
| Bruchzähigkeit[1] | DIN EN ISO 15732 | Single-edge-notch-bend[2] |
| Temperaturleitfähigkeitskoeffizient | DIN EN 821-2 | DIN V ENV 1159-2 |
| Spezifische Wärmekapazität | DIN EN 821-3 | DIN V ENV 1159-3 |
| 1: Die Bruchzähigkeit metallischer Werkstoffe wird gemäß DIN EN ISO 12737 bestimmt. 2: M. Kuntz. Risswiderstand keramischer Faserverbundwerkstoffe. Dissertation Universität Karlsruhe, Shaker Verlag, 1996. | | |

Der Wärmeleitfähigkeitskoeffizient ist über die folgende Beziehung definiert: Wärmeleitfähigkeitskoeffizient = Dichte x (spezifische Wärmekapazität) x Temperaturleitfähigkeitskoeffizient

Beispielhaft enthält die Tabelle 2 einen Vergleich zwischen den Eigenschaften monolithischer Keramiken und Faserverbundkeramiken auf der Basis von Aluminiumoxid.

Tabelle 2: Gegenüberstellung der physikalischen Eigenschaften von monolithischen Keramiken und Verbundkeramiken.

| Parameter | Monolithische Keramik Friatec Degussit® AL23 | Faserverbundkeramik WHIPOX® N610/45 |
|---|---|---|
| Offene Porosität in % | 0 | 26 |
| Dichte in $\frac{g}{cm^3}$ | 3,8 | 2,9 |
| E-Modul in *GPa* | 380 | 110 |
| Wärmeleitfähigkeitskoeffizient in $\frac{W}{m \cdot K}$ | 30 (@100° C) 5,5 (@1000° C) | 5,7 (@200° C) 2,7 (@1000° C) |

Nachteilig an der porösen Struktur von Faserverbundkeramiken ist ihre Untauglichkeit für die Herstellung von Druckapparaten mit einem Druck von > 0,5 bar. Ferner ist die schlechtere Wärmeleitfähigkeit im Vergleich zur unporösen monolithischen Keramik mit derselben chemischen Zusammensetzung nachteilig, d.h. wenn durch eine Schicht aus diesem Material ein Wärmestrom übertragen werden soll.

Die WO 2016/184776 A1 offenbart ein mehrschichtiges Verbundrohr beinhaltend eine Schicht aus unporöser monolithischer Oxidkeramik und eine Schicht aus oxidischer Faserverbundkeramik welches für die Herstellung von Reaktionsrohren einsetzbar ist, welche bei Betriebsdrücken von 1 bis 50 bar und Reaktionstemperaturen bis 1400° C betrieben werden und dabei von einer äußeren Wärmequelle - üblicherweise einer Heizkammer - stark erhitzt werden.

Allerdings können sich im Betrieb dieser Verbundrohre unerwünschte feste Ablagerungen an der Verbundrohrinnenwand bilden, wodurch der Wärmedurchgang verschlechtert wird und damit die Effektivität des Verfahrens so beeinträchtigt wird, dass der Ofen periodisch durch Freibrennen entkokt werden muss. In Extremfällen kann es dabei sogar zu einer vollständigen Verstopfung des freien Rohrquerschnitts im Innern des Verbundrohres kommen. Solche feste Ablagerungen können beispielsweise durch Nebenreaktionen von Kohlenwasserstoffen zum festen Kohlenstoff bei der Herstellung von Synthesegas durch Reformierung von Kohlenwasserstoffen mit Wasserdampf und / oder Kohlenstoffdioxid, bei der Koppelproduktion von Wasserstoff und Pyrolyse-Kohlenstoff durch die Pyrolyse von Kohlenwasserstoffen, bei der Herstellung von Blausäure aus Methan und Ammoniak oder aus Propan und Ammoniak, bei der Herstellung von Olefinen durch Wasserdampfspaltung von Kohlenwasserstoffen und/oder der Kupplung von Methan zu Ethylen, Acetylen und zu Benzolgebildet werden. Solche Kohlenstoffablagerungen werden weitläufig als Koks bezeichnet. Ihnen ist mit anderen industriellen Koksen gemeinsam, dass sie durch Hochtemperatur-Behandlung eines mindestens teilweise kohlenwasserstoffhaltigen Stoffes in sauerstoffarmer oder -freier Umgebung entstehen, wobei sauerstoffarm eine Umgebung bezeichnet, in der der vorhandene Sauerstoff nicht zur vollständigen Verbrennung zu CO2 und Wasserdampf ausreicht.

Im Falle der thermischen Dampfspaltung von Kohlenwasserstoffen werden drei verschiedene Arten von Koksen unterschieden - erstens, der sogenannte katalytische Koks, der sich an katalytisch wirkenden Elementen der Rohroberfläche, insbesondere Eisen (Fe) und Nickel (Ni) bildet, zweitens, der pyrolytische Koks, der durch Reaktionen in der Gasphase ohne Wechselwirkung mit der Rohrwand entsteht, und drittens, der Kondensationskoks, der durch auskondensieren höhermolekularer Kohlenwasserstoffe bei Temperaturen im Bereich 400 - 600 ° C auskondensiert und der insbesondere für den Austrittsbereich aus der Hochtemperaturzone relevant ist. Im Reaktionsrohr selbst dominieren die katalytische und pyrolytischen Verkokung.

Bereits seit den 60er Jahren wurden Ansätze zur Koks-Vermeidung in den Spaltrohren entwickelt. Dazu zählt die Entwicklung von hochlegierten, austenitischen metallischen Schleuderguss-Rohren, die unter Prozessbedingungen eine schützende Chrom- oder Aluminiumoxidschicht ausbilden sollen. Ein zweiter Entwicklungsstrang war die Reduzierung der Rohrwandtemperatur durch Verbesserung des rohrinnenseitigen Wärmeübergangs an das Prozessfluid. Diese Maßnahmen zielen darauf ab, die Temperatur an der Rohrwandinnenseite zu reduzieren und die dort stattfindende katalytische Verkokungsreaktion zu verlangsamen

Zur Verbesserung der Transporteigenschaften zwischen Gasstrom und Rohrwand sind im Stand der Technik vielfältige Konzepte offenbart. So gibt es beispielsweise Rohre mit längs der Achse verlaufenden Rippen oder eingeschobenen Strömungselementen.

In der WO 2015/052066 A1 ist ein Reaktionsrohr für die Herstellung von Cyanwasserstoff beschrieben, welches einen eingeschobenen rippenförmigen Einbaukörper aufweist. Hierdurch soll die Raum-Zeit-Ausbeute erhöht werden. Allerdings wird hierdurch der Gefahr von unerwünschten Ablagerungen an der Rohrwand nicht effektiv begegnet.

Die WO 2017/007649 A1 offenbart ein Reaktionsrohr mit Vertiefungen. Es werden generelle Ausführungen dazu offenbart und eine Vielzahl von Materialausgestaltungen, wobei sich hier jedoch kein Hinweis auf mehrschichtige Verbundrohre mit einem Wärmedurchgangskoeffizient von > 500 W/m$^2$/K findet, welches in seinem Aufbau über den Querschnitt der Wand des Verbundrohres als innere Schicht eine unporöse monolithische Oxid-Keramik aufweist, welche von einer äußeren Schicht aus oxidischer Faserverbundkeramik umschlossen wird und welches eine offene Porösität von 5% < ε < 50 % aufweist.

Die Implementierung von den erfindungsgemäßen Vertiefungen in solchen Verbundrohren hätte der Fachmann in Kenntnis der WO 2017/007649 A1 auch nicht in Erwägung gezogen, da die Einbringung von Vertiefungen in eine unporöse monolithische Oxidkeramik Grund für die Befürchtung bot, dass hierdurch die erforderliche Festigkeit des Bauteils nicht mehr gewährleistet wird. Somit stand zu befürchten, dass die erforderliche Festigkeit nur durch Erhöhung der Wandstärke erreicht werden kann, was den Vorteil der eingebrachten Vertiefungen aufgrund einer Verschlechterung des Wärmetransportes aufhebt.

Weiterhin hatte der Fachmann zu befürchten, dass durch die Einbringung von Vertiefungen bei diesem Werkstoff, welcher eine hohe Sprödigkeit aufweist, die Gefahr einer unerwünschten Rissbildung deutlich erhöht wird.

Und schließlich hätte der Fachmann keine Vertiefungen in keramische Rohre eingebracht, da hier das katalytische Kokswachstum ausgeschlossen ist und somit der von metallischen Rohren bekannte hohe Fertigungsaufwand nicht allein mit der Reduzierung des pyrolytischen Verkokens zu rechtfertigen ist.

Die WO 2017/178551 A1 beschreibt ebenfalls einen Reaktor für Spaltreaktionen bei welchem die innere Wand des Reaktorrohres Vertiefungen aufweist (Anspruch 1). Auch hier werden generelle Ausführungen dazu offenbart und eine Vielzahl von Materialausgestaltungen, wobei sich hier jedoch kein Hinweis auf mehrschichtige Verbundrohre mit einem Wärmedurchgangskoeffizient von > 500 W/m$^2$/K findet, welches in seinem Aufbau über den Querschnitt der Wand des Verbundrohres als innere Schicht eine unporöse monolithische Oxid-Keramik aufweist, welche von einer äußeren Schicht aus oxidischer Faserverbundkeramik umschlossen wird und welches eine offene Porösität von 5 < ε < 50 % aufweist. Die Implementierung von Vertiefungen in solchen Verbundrohren hätte der Fachmann in Kenntnis der WO 2017/178551 A1 auch nicht in Erwägung gezogen, da ihm ebenso wie in Kenntnis von WO2017/007649 eine unzureichender Festigkeit, zu hoher Sprödigkeit und zu hohen Fertigungswand Grund zur Befürchtung bot.

Die Aufgabe der vorliegenden Erfindung war demnach die Bereitstellung von Reaktionsrohren, die folgendes Eigenschaftsprofil aufweisen: (i) wärmedurchlässig mit einem Wärme-

$$durchgangskoeffizient > 500 \frac{W}{m^2\,K}$$ , (ii) temperaturbeständig bis >. 1100° C, (iii) druckbeständig bis ca. 5 bar bzw. beständig bei Druckdifferenzen bis ca. 5 bar, (iv) korrosionsbeständig gegen reduzierende und gegen oxidierende Atmosphäre mit einem Sauerstoffpartialdruck von 10$^{-25}$ bar bis 10 bar (v) temperaturwechselbeständig gemäß DIN EN 993-11 und (vi) chemisch inert gegenüber unerwünschte Ablagerungen, insbesondere inert gegenüber dem durch Metalle wie Eisen und Nickel katalysierten Verkoken an der Innenwand des Reaktionsrohres und (vii) darüber hinaus den Wärmetransport so verbessert, dass das pyrolytische Verkoken reduziert wird.

Hierbei wird ein mehrschichtiges Verbundrohr mit einem Wärmedurchgangskoeffizient von größer als 500 W/m$^2$/K beinhaltend mindestens zwei Schichten, welches in seinem Aufbau über den Querschnitt der Wand des Verbundrohres als innere Schicht eine unporöse monolithische Oxid-Keramik aufweist, welche von einer äußeren Schicht aus oxidischer Faserverbundkeramik umschlossen wird und welches eine offene Porösität von 5% < $\varepsilon$ < 50 % aufweist und welches an der inneren Oberfläche des Verbundrohres mehrere zur Außenwand des Verbundrohres gerichtete Vertiefungen aufweist, offenbart.

Die erfindungsgemäßen Vertiefungen können unregelmäßig oder bevorzugt regelmäßig auf der Innenwand des Verbundrohres angeordnet sein.

Die bevorzugte Anzahl der eingebrachten Vertiefungen auf eine spezifisches Flächenelement der Rohrinnenwand wird von den jeweiligen technischen Gegebenheiten beeinflusst. Generell empfiehlt es sich bevorzugt, dass die innere Oberfläche des Verbundrohres zu 10% bis 95, besonders bevorzugt 50% bis 90% mit den erfindungsgemäßen Vertiefungen versehen ist. Hierbei wird in Bezug auf die Vertiefungen auf den Flächenanteil Bezug genommen, den die Vertiefung direkt auf der Oberfläche der Rohrinnenwand jeweils einnimmt.

Die Form und die Tiefe der Vertiefungen kann gleich oder unterschiedlich über die Länge der Rohrinnenwand des Verbundrohres ausgestaltet sein. Es kann sich besonders empfehlen, die Form der erfindungsgemäßen Vertiefungen so zu gestalten, dass scharfe Kanten in der Kontur vermieden werden und stattdessen eine geschwungene, kurvenförmige Kontur gegeben ist, wie es z.B. bei spheroidischen, ovoiden, kugelförmigen, konkaven oder tränenförmigen Vertiefungen gegeben ist. Genauere Hinweise für eine mögliche Gestaltung solcher Vertiefungen kann der Fachmann der WO 2017/178551 A1 entnehmen.

Die erfindungsgemäßen Vertiefungen werden auf der Rohrinnenwand des Verbundrohres zur Außenseite hin gerichtet aufgebracht und befinden sich ausschließlich in der innersten Schicht des Verbundrohres, welche aus unporöser monolithischer Oxidkeramik besteht.

Die Implementierung von den erfindungsgemäßen Vertiefungen in solchen Verbundrohren hätte der Fachmann in Kenntnis der WO 2017/007649 A1 auch nicht in Erwägung gezogen, da die Einbringung von Vertiefungen in eine unporöse monolithische Oxidkeramik Grund für die Befürchtung bot, dass hierdurch die erforderliche Festigkeit des Bauteils nicht mehr gewährleistet wird. Somit stand zu befürchten, dass die erforderliche Festigkeit nur durch Erhöhung der Wandstärke erreicht werden kann, was den Vorteil der eingebrachten Vertiefungen aufgrund einer Verschlechterung des Wärmetransportes aufhebt.

Weiterhin hatte der Fachmann zu befürchten, dass durch die Einbringung von Vertiefungen bei diesem Werkstoff, welcher eine hohe Sprödigkeit aufweist, die Gefahr einer unerwünschten Rissbildung deutlich erhöht wird.

Und schließlich hätte der Fachmann keine Vertiefungen in keramische Rohre eingebracht, da hier das katalytische Kokswachstum ausgeschlossen ist und somit der von metallischen Rohren bekannte hohe Fertigungsaufwand nicht allein mit der Reduzierung des pyrolytischen Verkokens zu rechtfertigen ist.

Überraschenderweise ist es jedoch möglich, bei dem erfindungsgemäßen Verbundrohr die erforderlichen Eigenschaften zu erzielen. Hierbei ist es besonders vorteilhaft, wenn die maximale Tiefe der erfindungsgemäßen Vertiefungen 0,5 -2 mm beträgt. Wie bereits erwähnt kann die Tiefe der Vertiefungen ggf. innerhalb des Verbundrohres variieren. Dies kann sich besonders empfehlen, um die in Strömungsrichtung veränderliche Anforderungen hinsichtlich Wärmeübergang und Verkokungsneigung gezielt einzustellen. Die konkret bevorzugte Ausführung hängt von der gegebenen Ofengeometrie ab.

Weitere Hinweise für eine mögliche Ausgestaltung von solchen Vertiefungen kann der Fachmann der WO 2017/178551 A1 entnehmen.

Die Einbringung der erfindungsgemäßen Vertiefungen kann auf unterschiedliche Art und Weise erfolgen. Bevorzugt lassen sie sich vorteilhaft bei der Herstellung des monolithischen keramischen Rohres einprägen, indem sie nach den Bearbeitungsschritten des Extrudierens, Gießens oder Pressens und vor dem Brennen in den weichen Werkstoff eingebracht werden. Die Vertiefungen lassen sich vorteilhaft bei der Herstellung des monolithischen keramischen Rohres im Schritt der sogenannten Urformgebung durch Trocken-, Nass- oder isostatisches Pressen einprägen, weil hier die Formgebung und damit das Einbringen der Vertiefungen fertigungstechnisch einfach und mit großem geometrischem Freiheitsgrad möglich ist. Bevorzugt erfolgt die Einprägung der erfindungsgemäßen Vertiefungen bei der Urformgebung durch Pressverfahren.

Die Einprägung der erfindungsgemäßen Vertiefungen in das mehrschichtige Verbundrohr können insbesondere durch Press-Formgebung in verfahrenstechnisch einfacher und effektiver Art und Weise hergestellt werden. Vorteilhafterweise bestehen bei diesem Werkstoff in Vergleich zu metallischen Werkstoffen, die beispielsweise durch Schleuderguss oder Extrusion hergestellt werden, die Möglichkeit zur Formgebung im kalten Zustand (vor dem Brennen) und ohne Materialabtrag.

In Versuchen wurde überraschenderweise festgestellt, dass das erfindungsgemäße mehrschichtige Verbundrohr eine breitere Temperaturverteilung der Rohrinnenwand aufweist als ein Rohr gleicher Geometrie und Struktur basierend auf einem metallischen Werkstoff. Die niedrigere Temperatur führt dazu, dass die Verkokung bei dem erfindungsgemäßen Verbundrohr stärker unterbunden wird als bei einem vergleichbaren metallischen Rohr. Das hätte der Fachmann so nicht erwartet.

Die zwei Schichten bei dem erfindungsgemäßen Verbundrohr haften vorteilhaft aneinander durch kraftschlüssige oder stoffschlüssige Verbindungen. Relevante kraftschlüssige Verbindungen ist hier beispielsweise eine Pressverbindungen. Relevante stoffschlüssige Verbindungen für diese Erfindung sind Kleben und Sintern. Alle Verbindungsarten gehören zum Stand der Technik (W. Tochtermann, F. Bodenstein: Konstruktionselemente des Maschinenbaues, Teil 1. Grundlagen; Verbindungselemente; Gehäuse, Behälter, Rohrleitungen und Absperrvorrichtungen. Springer-Verlag, 1979).

Die Wand des mehrschichtigen Verbundrohrs beinhaltet vorteilhaft zumindest bereichsweise zwei Schichten, eine Schicht aus unporöser monolithischer Oxid-Keramik und eine Schicht aus oxidischer Faserverbundkeramik; d.h. es kann sich bei dem mehrschichtigen Verbundrohr auch um einen Verbundrohr-Abschnitt handeln. Beispielsweise sei ein zoniertes oder punktuelles nur bereichsweise aus zwei Schichten bestehendes Verbundrohr genannt. Bevorzugt beinhaltet jedoch die gesamte Wand des Verbundrohres, die einer äußeren Temperatur, z.B. durch eine Heizkammer, von > 1100° C ausgesetzt ist, mindestens zwei Schichten, eine Schicht aus unporöser monolithischer Oxid-Keramik und eine Schicht aus oxidischer Faserverbundkeramik.

Das mehrschichtige Verbundrohr weist in dem Rohrabschnitt, der einer äußeren Temperatur, z.B. durch eine Heizkammer, von > 1100° C ausgesetzt ist, vorteilhaft keine metallischen Schichten auf.

Vorteilhaft ist das Innenrohr umwickelt mit einer Schicht aus oxidischer Faserverbundkeramik. Die zwei Schichten können kraftschlüssig oder stoffschlüssig miteinander verbunden und ein Bauteil bilden. Die Eigenschaften dieses Bauteils sind durch die Temperaturbeständigkeit und das Verformungsverhalten der Schicht aus oxidischer Faserverbundkeramik bestimmt. Die Dichtigkeit ist durch das Innenrohr aus Oxid-Keramik gegeben. Bei Verwendung eines oxidkeramischen Innenrohrs weist die Innenseite der Rohrwand eine hohe chemische Beständigkeit und Abriebfestigkeit, mit einer Härte > 14000 MPa für Aluminiumoxid, > 12000 MPa für Zirkonoxid, auf.

Bei 1400° C sind beispielsweise Aluminiumoxid und Magnesiumoxid über den gesamten Bereich von einem Sauerstoffpartialdruck von $10^{-25}$ bar bis 10 bar beständig, während alle anderen keramischen Werkstoffe einen Übergang zwischen Reduktion und Oxidation durchlaufen und dadurch korrodieren (Darken, L. S., & Gurry, R. W. (1953). Physica/ chemistry of metals. McGraw-Hill).

Der Rohrinnendurchmesser des mehrschichtigen Verbundrohres beträgt vorteilhaft 10 mm bis 1000 mm, bevorzugt 10 mm bis 100 mm, insbesondere 40 mm bis 80 mm. Die gesamte Wandstärke aus mindestens zwei Schichten beträgt vorteilhaft 0,5 mm bis 50 mm, bevorzugt 1 mm bis 30 mm, insbesondere 2 mm bis 20 mm. Dabei beträgt vorteilhaft die Dicke der Schicht aus oxidischer Faserverbundkeramik weniger als 90 %, bevorzugt weniger als 50 %, insbesondere weniger als 25 % der gesamten Wandstärke; vorteilhaft beträgt die Dicke der Schicht aus oxidischer Faserverbundkeramik mindestens 10 % der gesamten Wandstärke. Die Dicke der Schicht aus monolithischer Oxid-Keramik beträgt vorteilhaft von 0,5 mm bis 45 mm, bevorzugt von 1 mm bis 25 mm, besonders bevorzugt von 2 mm bis 15 mm. Die Dicke der Schicht aus oxidischer Faserverbundkeramik beträgt vorteilhaft von 0,5 mm bis 5 mm, bevorzugt von 0,5 mm bis 3 mm.

Die Länge des mehrschichtigen Verbundrohres beträgt vorteilhaft 0,5 bis 20 m, bevorzugt 1 bis 10 m, insbesondere 1,5 bis 7 m. Es können mehrere solche Rohre durch Bögen und/oder Sammler miteinander verbunden sein, wobei ggf. auch diese Bögen und Sammler als mehrschichtige Verbundformteile ausgeführt sein können und die erfindungsgemäßen Vertiefungen aufweisen können.

Das offenbarte mehrschichtige Verbundrohr umfassend mindestens eine Schicht aus unporöser monolithischer Oxid-Keramik und mindestens eine Schicht aus oxidischer Faserverbundkeramik weist eine offene Porosität von 5% < ε < 50 %, bevorzugt 10% < ε < 30 % auf. Das mehrschichtige Verbundrohr ist besonders vorteilhaft gasdicht. Unter dem Begriff "gasdicht" wird ein Festkörper verstanden, der gemäß DIN EN 623-2 eine offene Porosität von Null besitzt. Die zulässige Messungenauigkeit liegt bei < 0,3 %.

Die Dichte der unporösen monolithischen Oxid-Keramik ist vorteilhaft größer als die Dichte der oxidischen Faserverbundkeramik. Die Dichte der unporösen monolithischen Oxid-Keramik beträgt vorteilhaft zwischen $1000\,\frac{kg}{m^3}$ und $7000\,\frac{kg}{m^3}$, insbesondere zwischen $2000\,\frac{kg}{m^3}$ und $5000\,\frac{kg}{m^3}$, beispielsweise $2800\,\frac{kg}{m^3}$ für Mullit (ca. 70% Aluminiumoxid) oder $3700\,\frac{kg}{m^3}$ für Aluminiumoxid mit einer Reinheit > 99,7%. Die Dichte der Schicht aus Faserverbundkeramik beträgt zwischen 500 $\frac{kg}{m^3}$ und 3000 $\frac{kg}{m^3}$. Das Verhältnis der Dichten der monolithischen Keramik und der Faserverbundkeramik in der Verbundstruktur beträgt vorteilhaft zwischen 1:1 und 3:1, insbesondere zwischen 1:1 und 2:1.

Der materialabhängige Elastizitätsmodul der unporösen monolithischen Oxid-Keramik ist vorteilhaft größer als der Elastizitätsmodul der oxidischen Faserverbundkeramik. Der Elastizitätsmodul der unporösen monolithischen Oxid-Keramik beträgt vorteilhaft zwischen 100 GPa und 500 GPa, insbesondere zwischen 150 GPa und 400 GPa, beispielsweise 150 GPa für Mullit (ca. 70% Aluminiumoxid) oder 380 GPa für Aluminiumoxid mit einer Reinheit > 99,7%. Der Elastizitätsmodul der Schicht aus Faserverbundkeramik beträgt zwischen 40 GPa und 200 GPa. Diese Werte gelten bei 25° C. Das Verhältnis der Elastizitätsmoduln der monolithischen Keramik und der Faserverbundkeramik in der Verbundstruktur beträgt vorteilhaft zwischen 1:1 und 5:1, insbesondere zwischen 1:1 und 3:1.

Der materialabhängige Wärmeleitfähigkeitskoeffizient der unporösen monolithischen OxidKeramik ist vorteilhaft größer als der Wärmeleitfähigkeitskoeffizient der oxidischen Faserverbundkeramik. Der Wärmeleitfähigkeitskoeffizient der unporösen monolithischen OxidKeramik beträgt vorteilhaft zwischen $1 \frac{W}{m \cdot K}$ und $50 \frac{W}{m \cdot K}$, insbesondere zwischen $2 \frac{W}{m \cdot K}$ und $40 \frac{W}{m \cdot K}$, beispielsweise $6 \frac{W}{m \cdot K}$ für Mullit (ca. 70% Aluminiumoxid) oder $30 \frac{W}{m \cdot K}$ für Aluminiumoxid mit einer Reinheit > 99,7%. Der Wärmleitfähigkeitskoeffizient der Schicht aus Faserverbundkeramik beträgt zwischen $0,5 \frac{W}{m \cdot K}$ und $10 \frac{W}{m \cdot K}$, bevorzugt zwischen $1 \frac{W}{m \cdot K}$ und $5 \frac{W}{m \cdot K}$. Diese Werte gelten bei 25° C. Das Verhältnis der Wärmeleitfähigkeitskoeffizienten der monolithischen Keramik und der Faserverbundkeramik in der Verbundstruktur beträgt vorteilhaft zwischen 1:1 und 10:1, insbesondere zwischen 1:1 und 5:1

Der Druckreaktor ist für folgende Druckbereiche ausgelegt; vorteilhaft 0,1 bar$_{abs}$ - 100 bar$_{abs}$, bevorzugt 1 bar$_{abs}$ - 10 bar$_{abs}$, weiter bevorzugt 1,5 bar$_{abs}$ - 5 bar$_{abs}$,

Die Druckdifferenz zwischen der Reaktionskammer und der Heizkammer beträgt vorteilhaft von 0 bar bis 100 bar, bevorzugt von 0 bar und 10 bar, weiter bevorzugt von 0 bar bis 5 bar,.

Bestimmung des Wärmedurchgangskoeffizientes Der Wärmedurchgangskoeffizient des erfindungsgemäßen mehrschichtigen Verbundrohres ist $> 500 \frac{W}{m^2 \, K}$, bevorzugt $> 1000 \frac{W}{m^2 \, K}$, weiter bevorzugt $> 2000 \frac{W}{m^2 \, K}$, insbesondere $> 3000 \frac{W}{m^2 \, K}$. Die Vorgehensweise zur Bestimmung des Wärmedurchgangskoeffizienten ist dem Fachmann bekannt (Kapitel Cb: Wärmedurchgang, VDI-Wärmeatlas, 8. Auflage, 1997). Gemäß dieser Definition gilt:

1997). Gemäß dieser Definition gilt:

$$k_{loc} = \frac{1}{R_w \cdot A}, \qquad R_w = \sum_{j=1}^{n} \left( \frac{\delta}{\lambda \cdot A_m} \right)_j$$

wobei

$$A_{m,j} = \left( \frac{A_1 - A_2}{\ln \frac{A_1}{A_2}} \right)_j$$

Dabei bedeuten die Symbole:

$R_w$: Wärmedurchgangswiderstand einer mehrschichtigen zylindrischen Wand in $\frac{K}{W}$,

$k_{loc}$: Wärmedurchgangskoeffizient einer mehrschichtigen zylindrischen Wand in $\frac{W}{m^2 \, K}$,

$A$: Zylindrische Mantelfläche in $m^2$,

$\lambda$: Wärmeleitfähigkeitskoeffizient einer homogenen Schicht in $\frac{W}{m \, K}$,

$\delta$: Dicke einer homogenen Schicht in m,

$n$: Anzahl der Schichten einer mehrschichtigen zylindrischen Wand,

die Indizes:

1: Innenseite einer zylindrischen Schicht,
2: Außenseite einer zylindrischen Schicht,
m: Mittlere Fläche

Das erfindungsgemäße mehrschichtige Verbundrohr kann über seine Länge einen variablen Querschnitt und eine variable Wandstärke haben. Beispielsweise kann das mehrschichtige Verbundrohr in Strömungsrichtung des Gases trichterförmig zu- oder abnehmen.

An den zwei Enden des mehrschichtigen Verbundrohres kann der Randbereich der äußeren Schicht vorteilhaft versiegelt sein. Die versiegelten Enden dienen als Übergänge zur gasdichten Verbindung des Verbundrohres mit metallischen gasführenden Leitungen, Verteilern, Sammlern oder Durchführungen durch die Hülle der umgebenden Heizkammer.

Als unporöse monolithische Oxid-Keramik können alle dem Fachmann bekannten oxidischen Keramiken verwendet werden, insbesondere analog dem Informationszentrum Technische Keramik (IZTK): Brevier technische Keramik. Fahner Verlag, Lauf (2003). Bevorzugt sind unporöse monolithische Oxid-Keramiken mit mindestens 99 Gew-% $Al_2O_3$ und/oder Mullit. Als unporöse Keramik können insbesondere Haldenwanger Pythagoras 1800Z™ (Mullit), Alsint 99,7™ oder Friatec Degussit® AL23 (Aluminiumoxid) zum Einsatz kommen.

Die Faserverbundwerkstoffe sind charakterisiert durch eine Matrix aus keramischen Partikeln zwischen die keramische Fasern, insbesondere Langfasern, als Wickelkörper oder als Textil eingebettet sind. Es wird von faserverstärkter Keramik, Verbundkeramik oder auch Faserkeramik gesprochen. Matrix und Faser können dabei im Prinzip aus allen bekannten keramischen Werkstoffen bestehen, wobei in diesem Zusammenhang auch Kohlenstoff als keramischer Werkstoff behandelt wird.

Unter "oxidischer Faserverbundkeramik" wird eine Matrix aus oxidischen keramischen Partikeln verstanden, die keramische, oxidische und/oder nicht-oxidische Fasern, enthält.

Bevorzugte Oxide der Fasern und/oder der Matrix sind Oxide eines Elementes aus der Gruppe: Be, Mg, Ca, Sr, Ba, Seltene Erden, Th, U, Ti, Zr, Hf, V, Nb, Ta, Cr, Mo, W, Mn, Fe, Co, Ni, Zn, B, Al, Ga, Si, Ge, Sn, Li, Na, K, Rb, Cs, Re, Ru, Os,Ir, Pt, Rh, Pd, Cu, Ag, Au, Cd, in, Tl, Pb, P, As, Sb, Bi, S, Se, Te, sowie Mischungen dieser Oxide.

Die Mischungen sind sowohl als Material für die Faser als auch für die Matrix vorteilhaft geeignet. Faser und Matrix müssen generell nicht aus demselben Material sein.

Grundsätzlich sind nicht nur binäre, sondern auch tertiäre und höhere Mischungen geeignet und von Bedeutung. In einer Mischung können die einzelnen Bestandteile in gleicher molarer Menge vorkommen, vorteilhaft sind aber Mischungen mit stark unterschiedlicher Konzentration der einzelnen Bestandteile der Mischung, bis hin zu Dotierungen, bei denen eine Komponente in Konzentrationen von < 1 % vorkommt.

Besonders vorteilhaft sind folgende Mischungen: Binäre und ternäre Mischungen aus Aluminiumoxid, Zirkoniumoxid und Yttriumoxid (z.B. Zirkoniumoxidverstärktes Aluminiumoxid); Mischungen aus Siliziumcarbid und Aluminiumoxid; Mischungen aus Aluminiumoxid und Magnesiumoxid (MgO-Spinell); Mischungen aus Aluminiumoxid und Siliziumoxid (Mullit); Mischung aus Aluminium- und Magnesiumsilikate, Ternäre Mischung aus Aluminiumoxid, Siliziumoxid und Magnesiumoxid (Cordierit); Steatit (Magnesiumsilikat); Zirkonoxid-verstärktes Aluminiumoxid; Stabilisiertes Zirkoniumoxid ($ZrO_2$): Stabilisatoren in Form von Magnesiumoxid (MgO), Calciumoxid (CaO) oder Yttriumoxid ($Y_2O_3$), gegebenenfalls kommen auch Ceroxid ($CeO_2$), Scandiumoxid ($ScO_3$) oder Ytterbiumoxid ($YbO_3$) als Stabilisatoren zum Einsatz; ferner Aluminiumtitanat (stöchiometrische Mischung von Aluminiumoxid und Titanoxid); Siliziumnitrid und Aluminiumoxid (Siliziumaluminiumoxinitride SIALON).

Als zirkoniumoxidverstärktes Aluminiumoxid wird vorteilhaft $Al_2O_3$ mit 10 bis 20 Mol-% $ZrO_2$ eingesetzt. Zur Stabilisierung von $ZrO_2$ kann vorteilhaft 10 bis 20 Mol-% CaO, bevorzugt 16 Mol-%, 10 bis 20 Mol-% MgO, bevorzugt 16, oder 5 bis 10 Mol-% $Y_2O_3$, bevorzugt 8 Mol-% ("vollstabilisiertes Zirkonoxid") oder 1 bis 5 Mol-% $Y_2O_3$, bevorzugt 4 Mol-% ("teilstabilisiertes Zirkonoxid") verwendet werden. Als ternäre Mischung sind z.B. 80% $Al_2O_3$, 18,4% $ZrO_2$ und 1,6% $Y_2O_3$ vorteilhaft.

Neben den genannten Materialien (Mischungen und Einzelbestandteile) sind auch Fasern aus Basalt, Bornitrid, Wolframcarbid, Aluminiumnitrid, Titandioxid, Bariumtitanat, Bleizirkonattitanat und/oder Borcarbid in oxid-keramischer Matrix denkbar.

Um eine gewünschte Armierung durch die zumindest zwei Schichten zu erzielen, können die Fasern der faserverstärkten Keramik radial umlaufend und/oder sich kreuzend auf der ersten Schicht der unporösen Keramik angeordnet sein.

Als Fasern kommen Verstärkungsfasern in Frage, die in die Klassen oxidische, carbidische, nitridische Fasern bzw. C-Fasern und SiBCN-Fasern fallen. Insbesondere ist vorgesehen, dass die Fasern des keramischen Verbundwerkstoffes Aluminiumoxid-, Mullit-, Siliziumcarbid-, Zirkonoxid- und/oder Kohlenstoff-Fasern sind. Mullit besteht dabei aus Mischkristallen aus Aluminiumoxid und Siliziumoxid. Bevorzugt ist die Verwendung von Fasern aus Oxidkeramik ($Al_2O_3$, $SiO_2$, Mullit) oder aus Nichtoxidkeramik (C, SiC).

Es kommen vorteilhaft kriechbeständige Fasern zum Einsatz, d. h. Fasern, die im Kriechbereich - im Temperaturbereich bis 1400 ° C - keine oder minimale zeitliche Zunahme der bleibenden Verformung, also der Kriechdehnung zeigen. Die Firma 3M gibt für die NEXTEL-Fasern folgende Grenztemperaturen an für die bleibende Dehnung von 1 % nach 1000 h unter Zugbelastung von 70 MPa an: NEXTEL 440: 875 ° C, NEXTEL 550 und NEXTEL 610: 1010 ° C, NEXTEL 720: 1120 ° C an (Referenz: Nextel™ Ceramic Textiles Technical Notebook, 3M, 2004).

Die Fasern haben vorteilhaft einen Durchmesser zwischen 10 und 12 $\mu$m. Sie sind vorteilhaft miteinander - üblicherweise mit Leinwand- oder Satinbindung - zu Textilbahnen verwoben, zu Schläuchen gestrickt oder als Faserbündel um eine Form gewickelt. Zur Herstellung des keramischen Verbundsystems werden die Faserbündel oder -gewebe beispielsweise mit einem Schlicker, der die Komponenten der späteren keramischen Matrix, vorteilhaft $Al_2O_3$ oder Mullit, enthält, infiltriert (Schmücker, M. (2007), Faserverstärkte oxidkeramische Werkstoffe, Materialwissenschaft und Werkstofftechnik, 38(9), 698-704). Durch Wärmebehandlung bei > 700 ° C entsteht schließlich eine hochfeste Verbundstruktur aus den Keramikfasern und der keramischen Matrix mit einer Zugfestigkeit von vorteilhaft > 50 MPa, bevorzugt > 70 MPa, weiter bevorzugt > 100MPa, insbesondere > 120MPa.

Bevorzugter Weise wird als keramischer Faserverbundwerkstoff SiC/ $Al_2O_3$, SiC/Mullit, C/ $Al_2O_3$, C/Mullit, $Al_2O_3$/$Al_2O_3$, $Al_2O_3$/Mullit, Mullit/ $Al_2O_3$ und/oder Mullit/Mullit eingesetzt. Dabei bezeichnet das Material vor dem Schrägstrich den Fasertyp und das Material nach dem Schrägstrich den Matrixtyp. Als Matrixsystem für die keramische Faserverbundstruktur können auch Siloxane, Si- Precursoren und unterschiedlichste Oxide, wie zum Beispiel auch Zirkonoxid, eingesetzt werden. Bevorzugt enthält der keramische Faserverbundwerkstoff mindestens 99 Gew-% $Al_2O_3$ und/oder Mullit.

In der vorliegenden Erfindung werden bevorzugt Faserverbundwerkstoffe auf der Basis oxidkeramischer Fasern, beispielsweise 3M™ NEXTEL™ 312, NEXTEL™ 440, NEXTEL™ 550, NEXTEL™ 610 oder NEXTEL™ 720 eingesetzt. Besonders bevorzugt ist die Verwendung von NEXTEL™ 610 und / oder NEXTEL™ 720.

Die Matrix weist einen Füllgrad an Fasern (Volumenanteil der Fasern in der Verbundstruktur) von 20 bis 40 % auf, der gesamte Feststoffgehalt der Verbundstruktur beträgt zwischen 50 und 80 %. Faserverbundkeramiken auf Basis oxidischer keramischer Fasern sind chemisch beständig in oxidierender und in reduzierender Gasatmosphäre (d. h. keine Gewichtsänderung nach Lagerung in Luft bei 1200 ° C über 15h (Referenz: Nextel™ Ceramic Textiles Technical Notebook, 3M, 2004)) und thermisch beständig bis über 1300 ° C. Faserverbundkeramiken besitzen ein quasi duktiles Verformungsverhalten. Damit sind sie temperaturwechselbeständig und besitzen ein quasi zähes Bruchverhalten. So kündigt sich das Versagen eines Bauteils an, bevor es bricht.

Der Faserverbundwerkstoff weist eine offene Porosität $\varepsilon$ von größer als 5 % bis kleiner als 50 %, bevorzugt von größer als 10% bis kleiner als 30% auf; sie ist demnach nicht gasdicht gemäß Definition in DIN 623-2.

Der Faserverbundwerkstoff weist vorteilhaft eine Langeinsatztemperatur von bis zu 1500 ° C, bevorzugt bis zu 1400 ° C, besonders bevorzugt bis zu 1300 ° C auf.

Der Faserverbundwerkstoff weist vorteilhaft eine Festigkeit > 50 MPa, bevorzugt > 70 MPa, besonders bevorzugt > 100 MPa, insbesondere > 120 MPa auf.

Der Faserverbundwerkstoff weist vorteilhaft eine Streckgrenze elastischer Verformung von 0,2 bis 1 % auf.

Der Faserverbundwerkstoff weist vorteilhaft eine Temperaturwechselbeständigkeit gemäß DIN EN 993-11 auf. Die Temperaturwechselbeständigkeit des erfindungsgemäßen Verbundrohres ist hierbei in der Regel größer als 50K/h, bevorzugt größer als 300K/h, besonders bevorzugt größer als 500K/h.

Die erfindungsgemäßen Vertiefungen weisen bevorzugt eine Tiefe von 0,5 bis 2mm auf.

Bevorzugt ist die innere Oberfläche des erfindungsgemäßen Verbundrohres zu 10% bis 95%, besonders bevorzugt zu 50% bis 90%, bezogen auf die gesamte innere Oberfläche des Verbundrohres mit Vertiefungen versehen.

Die Vertiefungen in dem erfindungsgemäßen Verbundroh sind in einer bevorzugten Ausführungsform in ihrem Querschnitt kreisförmig aufgebaut und weisen einen (maximalen) Durchmesser von 2mm bis 30mm auf.

Die innere Schicht des erfindungsgemäßen Verbundrohres weist eine Mindestschichtdicke von 0,5 mm bis 45 mm, bevorzugt von 1 mm bis 25 mm, besonders bevorzugt von 2 mm bis 15 mm auf.

Der Faserverbundwerkstoff weist vorteilhaft einen thermischen Wärmeausdehnungskoeffizient [ppm/K] von 4 bis 8,5 auf.

Der Faserverbundwerkstoff weist vorteilhaft eine thermische Leitfähigkeit von 0,5 bis $5\frac{W}{m\cdot K}$ auf.

Der keramische Faserverbundwerkstoff kann durch CVI (Chemical Vapour Infiltration) - Verfahren, Pyrolyse, insbesondere LPI (Liquid Polymer Infiltration) -Verfahren oder durch chemische Reaktion wie LSI (Liquid Silicon Infiltration) -Verfahren hergestellt werden.

Die Versiegelung beider Enden oder eines Endes des mehrschichtigen Verbundrohres kann auf vielfache Art und Weise durchgeführt werden:

Beispielsweise kann durch Infiltration oder Beschichtung der äußeren Schicht oder der inneren Schicht aus Faserverbundkeramik oder unporöser monolithischer Keramik mit einem Polymer, einer unporösen Keramik, Pyrokohlenstoff und/oder einem Metall eine Versiegelung erreicht werden. Die versiegelten Bereiche dienen als Dichtflächen. Diese Variante kann bis zu einem Temperaturbereich von < 400 ° C eingesetzt werden. Vorteilhaft wird das Verbundrohr lediglich im Randbereich zu dem metallischen Verbindungsstück beschichtet. "Randbereich" bedeutet der letzte Abschnitt vor dem Übergang zu einem anderen Werkstoff, bevorzugt zu einem metallischen Werkstoff, mit einer Länge entsprechend dem 0,05-bis 10-fachem Innendurchmesser des Verbundrohres, bevorzugt entsprechend dem 0,1- bis 5-fachem Innendurchmesser, insbesondere entsprechend dem 0,2- bis 2-fachem Innendurchmesser. Die Dicke der Imprägnierung entspricht vorteilhaft der kompletten Schichtdicke der Faserverbundkeramik im Randbereich. Die Verfahren zur Imprägnierung sind dem Fachmann bekannt.

Die vorliegende Erfindung umfasst demnach ein mehrschichtiges Verbundrohr beinhaltend mindestens zwei Schichten, eine Schicht aus unporöser monolithischer Keramik, bevorzugt Oxid-Keramik, und eine Schicht aus Faserverbundkeramik, bevorzugt oxidischer Faserverbundkeramik, wobei die äußere Schicht des Verbundrohres im Randbereich vor dem Übergang zu einem anderen Werkstoff, bevorzugt metallischen Werkstoff, mit Polymer, unporöser Keramik, (Pyro-)Kohlenstoff und/oder metallischem Werkstoff imprägniert oder beschichtet ist.

Eine andere Möglichkeit der Versiegelung besteht vorteilhaft in dem Anbringen einer Hülse aus Metall an den Randbereich des mehrschichtigen Verbundrohres, die bereichsweise mit Hilfe eines Überlappstoßes (5) zwischen der inneren und der äußeren Schicht angeordnet ist. Vorteilhaft enthält die Hülse aus Metall einen oder mehrere der folgenden Werkstoffe: Chrom, Titan, Molybdän, Nickelstahl 47Ni, Legierung 80Pt20Ir, Legierung 1.3981, Legierung 1.3917 oder ein Trimetall Kupfer / Invar / Kupfer. Vorteilhaft liegt das Verhältnis der Länge des Überlappstoßes (5) zu dem Innendurchmesser des Verbundrohres im Bereich von 0,05 bis 10, bevorzugt von 0,1 bis 5, insbesondere von 0,2 bis 2. In diesem Bereich ist die Hülse aus Metall mit der Außenseite der inneren Schicht gasdicht verbunden mit Fügetechniken, die dem Fachmann bekannt sind (Informationszentrum Technische Keramik (IZTK): Brevier technische Keramik, Fahner Verlag, Lauf (2003)). Die äußere Schicht ist durch eine stoffschlüssige Verbindung mit der Hülse aus Metall verbunden. Vorteilhaft beträgt die Länge des keramischen Überlappes, d.h. der Bereich beinhaltend äußere Schicht und metallische Hülse ohne innere Schicht, vom 0,05-fachen bis zum 10-fachen, bevorzugt von 0,1-fachen bis zum 5-fachen, insbesondere von 0,2-fachen bis zum 2-fachen des Innendurchmessers des Verbundrohres.

Die vorliegende Erfindung umfasst demnach ein mehrschichtiges Verbundrohr beinhaltend mindestens zwei Schichten, eine innere Schicht aus unporöser monolithischer Keramik, bevorzugt Oxid-Keramik, und eine äußere Schicht aus Faserverbundkeramik, bevorzugt oxidischer Faserverbundkeramik, wobei an der inneren Oberfläche des Verbundrohres mehrere zur Außenwand des Verbundrohres gerichtete Vertiefungen vorliegen und wobei eine Hülse aus Metall am Ende des Verbundrohres, die bereichsweise zwischen der inneren und der äußeren Schicht liegt, angeordnet ist.

Folglich umfasst die vorliegende Erfindung ein Verbindungsstück beinhaltend mindestens eine metallische gasführende Leitung, die in Längsrichtung des mehrschichtigen Verbundrohres, d.h. in Strömungsrichtung der Edukte, bereichsweise mit mindestens zwei keramischen Schichten überlappt, wobei zumindest eine keramische Schicht eine unporöse monolithische Keramik, bevorzugt Oxid-Keramik, beinhaltet und zumindest eine andere keramische Schicht eine Faserverbundkeramik, bevorzugt oxidischer Faserverbundkeramik, beinhaltet.

Folglich umfasst die vorliegende Erfindung eine Sandwichstruktur im Übergangsbereich zwischen metallischem Werkstoff und keramischem Werkstoff beinhaltend eine metallische Schicht, eine unporöse monolithische Keramik-Schicht, bevorzugt Oxid-Keramik, und Faserverbundkeramik-Schicht, bevorzugt oxidische Faserverbundkeramik. Vorteilhaft liegt die metallische Schicht zwischen der inneren und der äußeren keramischen Schicht.

Vorteilhaft umfasst die vorliegende Erfindung ein Verbindungsstück, das einen ersten RohrBereich beinhaltend ein metallisches Rohr, z. B. mindestens eine metallische gasführende Leitung, aufweist, das einen zweiten an den ersten Rohr-Bereich anschließenden Rohr-Bereich aufweist, der eine äußere Schicht aus Faserverbundkeramik und eine innere metallische Schicht aufweist und einen dritten an den zweiten Rohr-Bereich anschließenden RohrBereich aufweist, der eine Sandwichstruktur beinhaltend eine metallische Schicht, eine unporöse monolithische Keramik-Schicht und Faserverbundkeramik-Schicht aufweist und einen vierten an den dritten Rohr-Bereich anschließenden Rohr-Bereich aufweist, der ein mehrschichtiges Verbundrohr beinhaltend mindestens zwei Schichten, eine Schicht aus unporöser monolithischer Keramik und eine Schicht aus Faserverbundkeramik, aufweist. Vorteilhaft weist die Sandwichstruktur des Verbindungsstückes eine innere keramische Schicht, eine mittlere metallische Schicht und eine äußere keramische Schicht auf. Vorteilhaft ist die Faserverbundkeramik die äußere keramische Schicht. Vorteilhaft ist die unporöse monolithische Keramik-Schicht die innere Schicht. Alternativ ist die Faserverbundkeramik die innere keramische Schicht. Alternativ ist die unporöse monolithische Keramik-Schicht die äußere Schicht. Bevorzugt ist die Faserverbundkeramik oxidisch. Bevorzugt ist die unporöse monolithische Keramik eine Oxid-Keramik.

Die Länge des ersten Rohrbereichs ist größer als das 0,05-fache, bevorzugt größer als das 0,1-fache, insbesondere größer als das 0,2-fache des Innendurchmessers des mehrschichtigen Verbundrohres; vorteilhaft ist die Länge des ersten Rohrbereichs kleiner als 50 % der gesamten Länge des Verbundrohres.

Die Länge des zweiten Rohrbereichs beträgt vom 0,05-fachen bis zum 10-fachen, bevorzugt von 0,1-fachen bis zum 5-fachen, insbesondere von 0,2-fachen bis zum 2-fachen des Innendurchmessers des mehrschichtigen Verbundrohres.

Die Länge des dritten Rohrbereichs beträgt vom 0,05-fachen bis zum 10-fachen, bevorzugt von 0,1-fachen bis zum 5-fachen, insbesondere von 0,2-fachen bis zum 2-fachen des Innendurchmessers des Verbundrohres.

Vorteilhaft ist im dritten Rohrbereich die Wandstärke des metallischen Rohres, d.h. des metallischen Überlappes, das 0,01-fache bis 0,5-fache der gesamten Wandstärke, bevorzugt das 0,03-fache bis 0,3-fache der gesamten Wandstärke, insbesondere da 0,05-fache bis 0,1-fache der gesamten Wandstärke.

Vorteilhaft ist im zweiten Rohrbereich die Wandstärke des keramischen Überlappes, das 0,05- fache bis 0,9-fache der gesamten Wandstärke, bevorzugt das 0,05-fache bis 0,5-fache der gesamten Wandstärke, insbesondere das 0,05-fache bis 0,25-fache der gesamten Wandstärke. Vorteilhaft ist im zweiten Rohrbereich die Wandstärke der Hülse das 0,05-fache bis 0,9-fache der gesamten Wandstärke, bevorzugt das 0,05-fache bis 0,5-fache der gesamten Wandstärke, insbesondere das 0,05-fache bis 0,025fache der gesamten Wandstärke.

Die Dicke der Schicht aus monolithischer Keramik beträgt vorteilhaft von 0,5 mm bis 45 mm, bevorzugt von 1 mm bis 25 mm, besonders bevorzugt von 3 mm bis 15 mm. Die Dicke der Schicht aus oxidischer Faserverbundkeramik beträgt vorteilhaft von 0,5 mm bis 5 mm, bevorzugt von 0,5 mm bis 3 mm.

Eine andere Möglichkeit der Versiegelung besteht vorteilhaft in dem Anbringen einer Hülse aus Metall an das Ende des mehrschichtigen Verbundrohres, deren Innen- und Außenfläche bereichsweise mit der inneren und mit der äußeren Schicht verbunden ist. Die Verbindung mit der inneren Schicht ist gasdicht ausgeführt mit Fügetechniken, die dem Fachmann bekannt sind (Informationszentrum Technische Keramik (IZTK): Brevier technische Keramik, Fahner Verlag, Lauf (2003)). Die Verbindung mit der äußeren Schicht ist stoffschlüssig. Vorteilhaft umfasst die vorliegende Erfindung ein Verbindungsstück, das einen ersten RohrBereich beinhaltend ein metallisches Rohr, z. B. mindestens eine metallische gasführende Leitung, aufweist, das einen zweiten an den ersten Rohr-Bereich anschließenden Rohr-Bereich aufweist, der eine äußere keramische Schicht und eine innere metallische Schicht aufweist und einen dritten an den zweiten Rohr-Bereich anschließenden Rohr-Bereich aufweist, der eine Sandwichstruktur beinhaltend eine innere metallische Schicht, eine mittlere und eine äußere Keramik-Schicht, wobei eine der Keramik-Schichten eine unporöse monolithische Keramik-Schicht und die andere Keramik-Schicht eine Faserverbundkeramik-Schicht aufweist und einen vierten an den dritten Rohr-Bereich anschließenden Rohr-Bereich aufweist, der ein mehrschichtiges Verbundrohr beinhaltend mindestens zwei Schichten, eine Schicht aus unporöser monolithischer Keramik und eine Schicht aus Faserverbundkeramik, aufweist.

Vorteilhaft ist die Faserverbundkeramik die äußere keramische Schicht. Vorteilhaft ist die unporöse monolithische Keramik-Schicht die innere Schicht. Alternativ ist die Faserverbundkeramik die innere keramische Schicht. Alternativ ist die unporöse monolithische Keramik-Schicht die äußere Schicht. Bevorzugt ist die Faserverbundkeramik oxidisch. Bevorzugt ist die unporöse monolithische Keramik eine Oxid-Keramik.

Die Länge des ersten Rohrbereichs ist größer als das 0,05-fache, bevorzugt größer als das 0,1-fache, insbesondere größer als das 0,2-fache des Innendurchmessers des mehrschichtigen Verbundrohres; vorteilhaft ist die Länge des ersten Rohrbereichs kleiner als 50 % der gesamten Länge des Verbundrohres.

Die Länge des zweiten Rohrbereichs beträgt vom 0,05-fachen bis zum 10-fachen, bevorzugt von 0,1-fachen bis zum 5-fachen, insbesondere von 0,2-fachen bis zum 2-fachen des Innendurchmessers des mehrschichtigen Verbundrohres.

Die Länge des dritten Rohrbereichs beträgt vom 0,05-fachen bis zum 10-fachen, bevorzugt von 0,1-fachen bis zum 5-fachen, insbesondere von 0,2-fachen bis zum 2-fachen des Innendurchmessers des Verbundrohres.

Vorteilhaft ist im dritten Rohrbereich die Wandstärke des metallischen Rohres, d. h. des metallischen Überlappes, das 0,01-fache bis 0,5-fache der gesamten Wandstärke, bevorzugt das 0,03-fache bis 0,3-fache der gesamten Wandstärke, insbesondere da 0,05-fache bis 0,1-fache der gesamten Wandstärke.

Vorteilhaft ist im zweiten Rohrbereich die Wandstärke des keramischen Überlappes, das 0,1-fache bis 0,95-fache der gesamten Wandstärke, bevorzugt das 0,5-fache bis 0,95-fache der gesamten Wandstärke, insbesondere das 0,8-fache bis 0,95-fache der gesamten Wandstärke. Vorteilhaft ist im zweiten Rohrbereich die Wandstärke der Hülse, das 0,05-fache bis 0,9-fache der gesamten Wandstärke, bevorzugt das 0,05-fache bis 0,5-fache der gesamten Wandstärke, insbesondere das 0,05-fache bis 0,2-fache der gesamten Wandstärke.

Die Dicke der Schicht aus monolithischer Keramik beträgt vorteilhaft von 0,5 mm bis 45 mm, bevorzugt von 1 mm bis 25 mm, besonders bevorzugt von 3 mm bis 15 mm. Die Dicke der Schicht aus oxidischer Faserverbundkeramik beträgt vorteilhaft von 0,5 mm bis 5 mm, bevorzugt von 0,5 mm bis 3 mm.

Vorteilhaft umfasst die vorliegende Erfindung ein Verbindungsstück, das einen ersten RohrBereich beinhaltend ein metallisches Rohr, z. B. mindestens eine metallische gasführende Leitung, aufweist, das einen zweiten an den ersten Rohr-Bereich anschließenden Rohr-Bereich aufweist, der eine Sandwichstruktur beinhaltend eine innere Keramik-Schicht, eine mittlere metallische Schicht und eine äußere Keramik-Schicht, wobei eine der Keramik-Schichten eine unporöse monolithische Keramik-Schicht und die andere Keramik-Schicht eine Faserverbundkeramik-Schicht aufweist und einen dritten an den zweiten Rohr-Bereich anschließenden Rohr-Bereich aufweist, der ein mehrschichtiges Verbundrohr beinhaltend mindestens zwei Schichten, eine Schicht aus unporöser monolithischer Keramik und eine Schicht aus Faserverbundkeramik, aufweist.

Vorteilhaft ist die Faserverbundkeramik die innere keramische Schicht. Vorteilhaft ist die unporöse monolithische Keramik-Schicht die äußere Schicht. Alternativ ist die Faserverbundkeramik die äußere keramische Schicht. Alternativ ist die unporöse monolithische Keramik-Schicht die innere Schicht. Bevorzugt ist die Faserverbundkeramik oxidisch. Bevorzugt ist die unporöse monolithische Keramik eine Oxid-Keramik.

Die Länge des zweiten Rohrbereichs beträgt vom 0,05-fachen bis zum 10-fachen, bevorzugt von 0,1-fachen bis zum 5-fachen, insbesondere von 0,2-fachen bis zum 2-fachen des Innendurchmessers des mehrschichtigen Verbundrohres.

Vorteilhaft ist im zweiten Rohrbereich die Wandstärke des metallischen Rohres, d.h. des metallischen Überlappes, das 0,01-fache bis 0,5-fache der gesamten Wandstärke, bevorzugt das 0,03-fache bis 0,3-fache der gesamten Wandstärke, insbesondere das 0,05-fache bis 0,1-fache der gesamten Wandstärke.

Vorteilhaft ist im zweiten Rohrbereich die Wandstärke des keramischen Überlappes, das 0,1-fache bis 0,95-fache der gesamten Wandstärke, bevorzugt das 0,5-fache bis 0,95-fache der gesamten Wandstärke, insbesondere das 0,8-fache bis 0,95-fache der gesamten Wandstärke. Vorteilhaft ist im zweiten Rohrbereich die Wandstärke der Hülse, das 0,05-fache bis 0,9-fache der gesamten Wandstärke, bevorzugt das 0,05-fache bis 0,5-fache der gesamten Wandstärke, insbesondere das 0,05-fache bis 0,2-fache der gesamten Wandstärke.

Die Dicke der Schicht aus monolithischer Keramik beträgt vorteilhaft von 0,5 mm bis 45 mm, bevorzugt von 1 mm bis 25 mm, besonders bevorzugt von 3 mm bis 15 mm. Die Dicke der Schicht aus oxidischer Faserverbundkeramik beträgt vorteilhaft von 0,5 mm bis 5 mm, bevorzugt von 0,5 mm bis 3 mm.

Vorteilhaft umfasst die vorliegende Erfindung ein Verbindungsstück, das einen ersten RohrBereich beinhaltend ein metallisches Rohr, z. B. mindestens eine metallische gasführende Leitung, aufweist, das einen zweiten an den ersten Rohr-Bereich anschließenden Rohr-Bereich aufweist, der eine Sandwichstruktur beinhaltend eine innere und eine mittlere Keramik-Schicht und eine äußere metallische Schicht, wobei eine der Keramik-Schichten eine unporöse monolithische Keramik-Schicht und die andere Keramik-Schicht eine Faserverbundkeramik-Schicht aufweist und einen dritten an den zweiten Rohr-Bereich anschließenden Rohr-Bereich aufweist, der ein mehrschichtiges Verbundrohr beinhaltend mindestens zwei Schichten, eine Schicht aus unporöser monolithischer Keramik und eine Schicht aus Faserverbundkeramik, aufweist.

Vorteilhaft ist die Faserverbundkeramik die innere keramische Schicht. Vorteilhaft ist die unporöse monolithische Keramik-Schicht die äußere Schicht. Alternativ ist die Faserverbundkeramik die äußere keramische Schicht. Alternativ ist die unporöse monolithische Keramik-Schicht die innere Schicht. Bevorzugt ist die Faserverbundkeramik oxidisch. Bevorzugt ist die unporöse monolithische Keramik eine Oxid-Keramik.

Die Länge des zweiten Rohrbereichs beträgt vom 0,05-fachen bis zum 10-fachen, bevorzugt von 0,1-fachen bis zum 5-fachen, insbesondere von 0,2-fachen bis zum 2-fachen des Innendurchmessers des mehrschichtigen Verbundrohres.

Vorteilhaft ist im zweiten Rohrbereich die Wandstärke des metallischen Rohres, d.h. des metallischen Überlappes, das 0,01-fache bis 0,5-fache der gesamten Wandstärke, bevorzugt das 0,03-fache bis 0,3-fache der gesamten Wandstärke, insbesondere da 0,05-fache bis 0,1-fache der gesamten Wandstärke.

Vorteilhaft ist im zweiten Rohrbereich die Wandstärke des keramischen Überlappes, das 0,1-fache bis 0,95-fache der gesamten Wandstärke, bevorzugt das 0,5-fache bis 0,95-fache der gesamten Wandstärke, insbesondere da 0,8-fache bis 0,95-fache der gesamten Wandstärke. Vorteilhaft ist im zweiten Rohrbereich die Wandstärke der Hülse, das 0,05-fache bis 0,9-fache der gesamten Wandstärke, bevorzugt das 0,05-fache bis 0,5-fache der gesamten Wandstärke, insbesondere das 0,05-fache bis 0,2-fache der gesamten Wandstärke.

Die Dicke der Schicht aus monolithischer Keramik beträgt vorteilhaft von 0,5 mm bis 45 mm, bevorzugt von 1 mm bis 25 mm, besonders bevorzugt von 2 mm bis 15 mm. Die Dicke der Schicht aus oxidischer Faserverbundkeramik beträgt vorteilhaft von 0,5 mm bis 5 mm, bevorzugt von 0,5 mm bis 3 mm.

Das mehrschichtige Verbundrohr ist typischerweise vertikal angeordnet, an einem Ende fest und am anderen Ende lose gelagert. Vorzugsweise ist es am unteren Ende fest eingespannt und am oberen Ende in axialer Richtung verschiebbar geführt. In dieser Anordnung kann sich das Rohr spannungsfrei thermisch ausdehnen.

Eine Variante der Lösung besteht aus zwei konzentrischen Rohren. Das innere Rohr weist vorteilhaft einen Rohrinnendurchmesser von 10 mm bis 100 mm, bevorzugt 15 mm bis 50 mm, insbesondere 20 mm bis 30 mm, auf. Das innere Rohr ist vorteilhaft auf beiden Seiten offen und das äußere Rohr vorteilhaft einseitig verschlossen. Das äußere Rohr weist vorteilhaft einen Rohrinnendurchmesser von 20 mm bis 1000 mm, bevorzugt 50 mm bis 800 mm, insbesondere 100 mm bis 500 mm, auf. Am offenen Randbereich sind die Wände des inneren und des äußeren Rohres vorteilhaft versiegelt. Die Hauptreaktionsstrecke befindet sich vorteilhaft im Ringraum zwischen dem Innen- und dem Außenrohr. Dabei können die Reaktanden entweder in den Ringraum eingeleitet und der Produktstrom aus dem Innenrohr abgezogen werden oder umgekehrt. Die Anschlüsse für den Zulauf und den Ablauf befinden sich am offenen Rohrende. Das geschlossene Rohrende kann lose (ohne jede Führung) in den Heizraum ragen und dort ungehindert expandieren. Dadurch können in axialer Richtung keine temperaturinduzierte Spannungen entstehen. Durch diese Konfiguration wird gewährleistet, dass die mehrschichtigen Verbundrohre nur einseitig, im Kalten eingespannt und abgedichtet werden müssen und am geschlossenen Ende ungehindert die thermische Ausdehnung eingehen können.

Die vorliegende Erfindung umfasst somit einen Doppelrohr-Reaktor für endotherme Reaktionen, der dadurch gekennzeichnet ist, dass der Reaktor zwei mehrschichtige Verbundrohre mit einem Wärmedurchgangskoeffizient von > 500 W/m$^2$/K beinhaltend mindestens jeweils zwei Schichten, eine Schicht aus unporösen monolithischem Keramik und eine Schicht aus Faserverbundkeramik aufweist, wobei das eine Verbundrohr das andere Verbundrohr umschließt und das innere Verbundrohr auf beiden Seiten offen ist und das äußere Rohr einseitig verschlossen ist.

Vorteilhaft ist die Faserverbundkeramik die äußere keramische Schicht des mehrschichtigen Verbundrohres beinhaltend zwei konzentrischen Rohre. Vorteilhaft ist die unporöse monolithische Keramik-Schicht die innere Schicht. Alternativ ist die Faserverbundkeramik die innere keramische Schicht. Alternativ ist die unporöse monolithische Keramik-Schicht die äußere Schicht. Bevorzugt ist die Faserverbundkeramik oxidisch. Bevorzugt ist die unporöse monolithische Keramik eine Oxid-Keramik.

Durch den doppelschichtigen Aufbau kann die Dichtigkeit und Temperaturbeständigkeit eines Rohres aus monolithischer unporöser Keramik mit dem gutmütigen Versagensverhalten der Faserverbundkeramik ("Riss vor Bruch") kombiniert werden. Die erfindungsgemäße Vorrichtung mit versiegelten Randbereichen ermöglicht die gasdichte Anbindung der mehrschichtigen Verbundrohre an die konventionell ausgeführte Peripherie.

Vorteilhaft werden die erfindungsgemäßen keramischen mehrschichtigen Verbundrohre für folgende Prozesse eingesetzt:

- Herstellung von Synthesegas durch Reformierung von Kohlenwasserstoffen mit Wasserdampf und / oder $CO_2$.
- Koppelproduktion von Wasserstoff und Pyrolyse-Kohlenstoff durch die Pyrolyse von Kohlenwasserstoffen.
- Herstellung von Blausäure aus Methan und Ammoniak (Degussa) oder aus Propan und Ammoniak.
- Herstellung von Olefinen durch Wasserdampfspaltung von Kohlenwasserstoffen (Naphtha, Ethan, Propan).
- Kupplung von Methan zu Ethylen, Acetylen und zu Benzol.

Vorteilhaft werden die erfindungsgemäßen keramischen Verbundrohre als Reaktionsrohre in folgenden Anwendungen eingesetzt:

- Reaktoren mit axialer Temperaturkontrolle, wie

    o Wirbelschichtreaktoren,
    o Rohrbündelreaktoren,
    o Reformer- und Spaltöfen.

- Strahlrohre, Flammrohre.
- Gegenstromreaktoren.
- Membranreaktoren.
- Drehrohre für Drehrohröfen.

Im folgenden werden die Vorteile des erfindungsgemäßen mehrschichtigen Verbundrohres durch Vergleichsbeispiele belegt.

Beispiel 1: Vergleich der Temperaturverteilung auf einem erfindungsgemäßen mehrschichtigen Verbundrohr mit Vertiefungen zu einem mehrschichtigen Verbundrohr ohne Vertiefungen

Durch numerische Simulation (CFD = computational fluid dynamics) wurde die Temperaturverteilung in einem dampfdurchströmten Rohr berechnet. In diesem Beispiel wurde ein 1* m langes mehrschichtiges keramisches Verbundrohr mit 0,047 m Innendurchmesser und Rohrwandstärken von 4 mm der monolithischen Keramik und 1,5 mm Faserkeramik simuliert.

Die folgende Tabelle 3 zeigt die Eigenschaften der hier zur Anwendung kommenden Rohrwerkstoffe.

| Stoffdaten bei 900 ° C | $Al_2O_3$ | Faserkeramik | Metallrohr |
|---|---|---|---|
| $\rho$ (Dichte, kg/m3) | 2800 | 2900 | 7600 |
| $c_p$ (spez. Wärmekapazität, J/kgK) | 900 | 900 | 663 |
| $\lambda$ (Wärmeleitfähigkeit, W/mK) | 706,1*T'$^{(-0,672)}$ | 58,9*T'$^{(-0,479)}$ | 24 |
| T' = lokale Temperatur in ° C | | | |

Neben einem Rohr mit erfindungsgemäßen Vertiefungen wurde ein Rohr ohne Vertiefungen gleicher Struktur simuliert. In dem Rohr mit Vertiefungen wurden 8 Vertiefungen pro Umfangssegment mit einem Radius von jeweils 13,8 mm und eine versetzte Anordnung in axiale Richtung mit einem Abstand von 12,5 mm zwischen den Zentren der Vertiefungen abgebildet.

In der Simulation wurde eine Eintrittstemperatur des Fluids von 750 ° C, ein Massenstrom von 8 kg/s und eine konstante äußere Rohrwandtemperatur von 950 ° C vorgegeben.

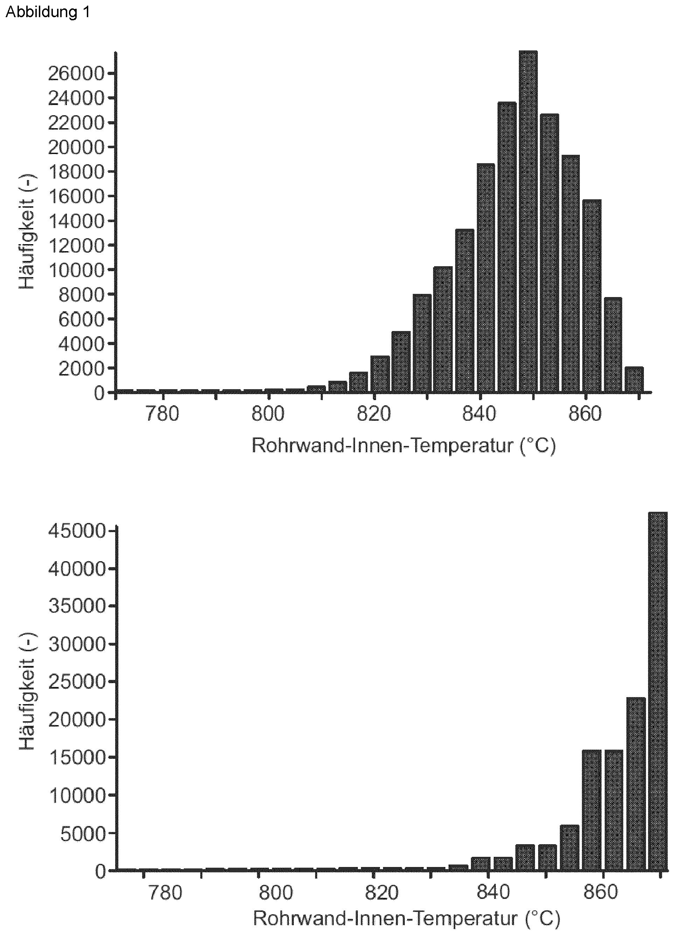

Die Ergebnisse der Simulation sind in Abbildung 1 dargestellt. Es ist die Häufigkeitsverteilung (Anzahl der in der Simulation diskretisierten Oberflächenelemente) über Rohrwand-Innen-Temperatur aufgetragen, im linken Schaubild für das erfindungsgemäße Rohr mit Vertiefungen und im rechten Schaubild für ein Rohr gleichen Aufbaus ohne Vertiefungen. Es ist zu sehen, dass die Vertiefungen die mittlere Rohrwandtemperatur und damit die Verkokung gegenüber dem Rohr ohne Vertiefungen insgesamt reduzieren und gleichzeitig der auf den Fluidstrom übertragene Wärmestrom aufgrund des besseren Wärmeübergangs um 14% steigt. Außerdem zeigt das Beispiel, dass die Verteilung der Rohrwandtemperatur durch den lokal besseren Wärmeübergang an den Vertiefungen breiter wird. Dies ist insbesondere deshalb vorteilhaft, weil dieser Effekt die Verkokung im Innern der Vertiefungen reduziert und damit die Struktur der Vertiefungen und der Effekt der besseren Wärmeübertragung auch während des Verkokungsprozesses erhalten bleibt.

Beispiel 2: Vergleich der Temperaturverteilung auf einem erfindungsgemäßen mehrschichtigen Verbundrohr mit Vertiefungen zu einem metallischen Rohr (Werkstoff S+C Centralloy® HT-E) mit Vertiefungen

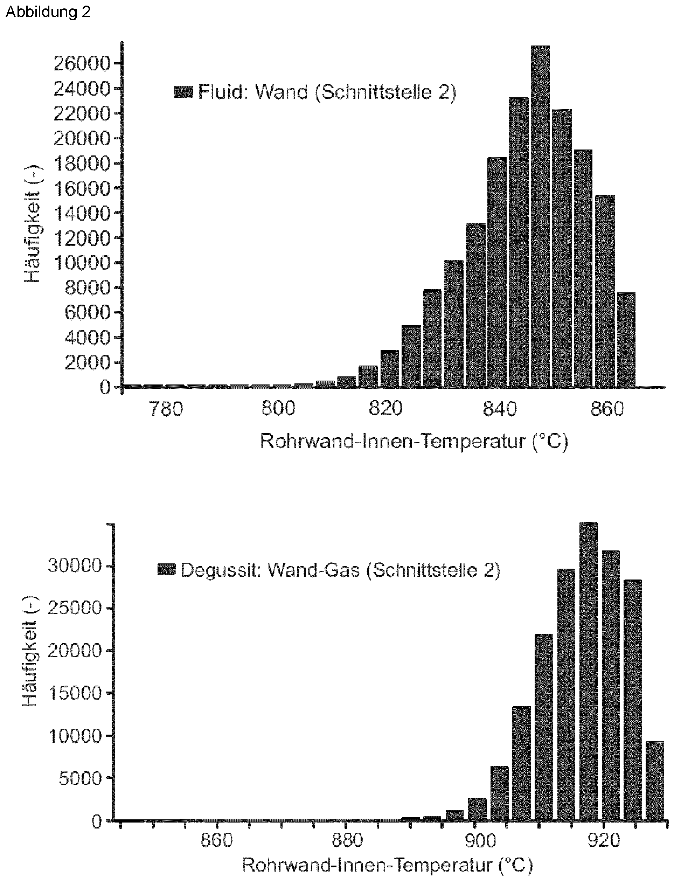

In einem zweiten Beispiel wurde obiges erfindungsgemäße mehrschichtige Verbundrohr mit Vertiefungen mit einem geometrisch identischen metallischen Rohr mit Vertiefungen verglichen. Die Ergebnisse der Simulation sind in Abbildung 2 dargestellt. Es ist die Häufigkeitsverteilung (Anzahl der in der Simulation diskretisierten Oberflächenelemente) über Rohrwand-Innen-Temperatur aufgetragen, im linken Schaubild für das erfindungsgemäße Rohr mit Vertiefungen und im rechten Schaubild für ein metallisches Rohr gleicher Struktur ebenfalls mit Vertiefungen gleicher Struktur. Wie Abbildung 2 zeigt, ist die Temperaturverteilung beim keramischen Rohr breiter. Das spiegelt einen größeren Temperaturunterschied zwischen den Vertiefungen (geringe Temperatur) und der restlicher Rohrwandoberfläche (hohe Temperatur) beim keramischen Rohr wider. Es wird vermutet, dass die schlechtere Wärmeleitung im keramischen Rohr zu dieser stärkeren Temperaturstreuung führt. Dieses Ergebnis ist überraschend und zeigt, dass die Vertiefungen bei einem keramischen Rohr vorteilhafter als bei metallischen Rohren sind, da bei einem keramischen Rohr die Koksbildung insbesondere an den Vertiefungen reduziert wird und somit der positive Effekt der Vertiefungen länger erhalten bleibt. Bei metallischen Rohren werden die Vertiefungen schnell durch Koksbildung ausgefüllt.

## Patentansprüche

1. Mehrschichtiges Verbundrohr mit einem Wärmedurchgangskoeffizient, gemäss der in der Beschreibung angedeuteten Bestimmung des Wärmedurchgangskoeffizientes, von > 500 W/m$^2$/K beinhaltend mindestens zwei Schichten, welches in seinem Aufbau über den Querschnitt der Wand des Verbundrohres als innere Schicht eine nicht offen poröse monolithische Oxid-Keramik aufweist, welche von einer äußeren Schicht aus oxidischer Faserverbundkeramik umschlossen wird, wobei diese äußere Schicht eine offene Porösität ε gemäss DIN V ENV 1389 von 5% < ε < 50 %, bevorzugt 10%< ε< 30 %, aufweist und welches an der inneren Oberfläche des Verbundrohres mehrere zur Außenwand des Verbundrohres gerichtete Vertiefungen aufweist.

2. Verbundrohr nach Anspruch 1, **dadurch gekennzeichnet, dass** die Temperaturwechselbeständigkeit im Sinne von DIN EN 993-11 des Verbundrohres größer als 50 K/h ist.

3. Verbundrohr nach mindestens einem der Ansprüche 1 bis 2, **dadurch gekennzeichnet, dass** die Vertiefungen eine Tiefe von 0,5mm bis 2mm aufweisen.

4. Verbundrohr nach mindestens einem der Ansprüche 1 bis 3, **dadurch gekennzeichnet, dass** die Vertiefungen gleichmäßig über die innere Oberfläche des Verbundrohres verteilt sind.

5. Verbundrohr nach mindestens einem der Ansprüche 1 bis 4, **dadurch gekennzeichnet, dass** die Vertiefungen ungleichmäßig über die innere Oberfläche des Verbundrohres verteilt sind.

6. Verbundrohr nach mindestens einem der Ansprüche 1 bis 5, **dadurch gekennzeichnet, dass** die innere Oberfläche des Verbundrohres zu 10% bis 95% bezogen auf die gesamte innere Oberfläche des Verbundrohres mit Vertiefungen versehen ist.

7. Verbundrohr nach mindestens einem der Ansprüche 1 bis 6, **dadurch gekennzeichnet, dass** die Vertiefungen konkav ausgeführt sind.

8. Verbundrohr nach mindestens einem der Ansprüche 1 bis 7, **dadurch gekennzeichnet, dass** die Vertiefungen in ihrem Querschnitt kreisförmig aufgebaut sind und einen Durchmesser von 2mm bis 30mm aufweisen.

9. Verbundrohr nach mindestens einem der Ansprüche 1 bis 8, **dadurch gekennzeichnet, dass** die gesamte Wandstärke des Verbundrohres 0,5 mm bis 50 mm beträgt.

10. Verbrundrohr nach mindestens einem der Ansprüche 1 bis 9, **dadurch gekennzeichnet, dass** der Rohrinnendurchmesser des Verbundrohres 10 mm bis 1000 mm beträgt.

11. Verbundrohr nach mindestens einem der Ansprüche 1 bis 10, **dadurch gekennzeichnet, dass** als oxidische Faserverbundkeramik $SiC/Al_2O_3$, SiC/Mullit, $C/Al_2O_3$, C/Mullit, $Al_2O_3/Al_2O_3$, $Al_2O_3$/Mullit, Mullit/$Al_2O_3$ und/oder Mullit/Mullit eingesetzt wird.

12. Verbundrohr nach mindestens einem der Ansprüche 1 bis 11, **dadurch gekennzeichnet, dass** das Verbundrohr zwei Schichten, eine innere und eine äußere Schicht beinhaltet, wobei die innere Schicht aus unporöser monolithischer Oxidkeramik aufgebaut ist und die äußere Schicht aus oxidischer Faserverbundkeramik aufgebaut ist.

13. Verbundrohr nach mindestens einem der Ansprüche 1 bis 11, **dadurch gekennzeichnet, dass** das Verbundrohr eine Struktur aufweist, in welcher die unporöse monolithische Oxid Keramik von oxidischer Faserverbundkeramik bedeckt wird.

14. Verbundrohr nach mindestens einem der Ansprüche 1 bis 13, **dadurch gekennzeichnet, dass** die innere Schicht eine Mindestschichtdicke von 0,5 mm bis 45 mm aufweist.

15. Verwendung des Verbundrohres nach mindestens einem der Ansprüche 1 bis 14 bei der Herstellung von Synthesegas durch Reformierung von Kohlenwasserstoffen mit Wasserdampf und / oder Kohlenstoffdioxid, Koppelproduktion von Wasserstoff und Pyrolyse-Kohlenstoff durch die Pyrolyse von Kohlenwasserstoffen, Herstellung von Blausäure aus Methan und Ammoniak oder aus Propan und Ammoniak, Herstellung von Olefinen durch Wasserdampfspaltung von Kohlenwasserstoffen und/oder Kupplung von Methan zu Ethylen, Acetylen und zu Benzol.

16. Verwendung des Verbundrohres nach mindestens einem der Ansprüche 1 bis 14 als Reaktionsrohre in Reaktoren mit axialer Temperaturkontrolle, Gegenstromreaktoren, Membranreaktoren, Strahlrohre, Flammrohre, und/oder Drehrohre für Drehrohröfen.

17. Verfahren zur Herstellung des mehrschichtigen Verbundrohres gemäß Anspruch 1, **dadurch gekennzeichnet, dass** man die Vertiefungen durch Pressverfahren einprägt.

**Claims**

1. A multilayered composite tube having a heat transfer coefficient, according to the determination of heat transfer coefficient specified in the description, of > 500 W/m$^2$/K comprising at least two layers which in its construction over the cross section of the wall of the composite tube comprises as an inner layer a zero-open-porosity monolithic oxide ceramic surrounded by an outer layer of oxidic fiber composite ceramic, wherein this outer layer has an open porosity $\varepsilon$ according to DIN V ENV 1389 of 5% < $\varepsilon$ < 50 %, preferably 10% < $\varepsilon$ < 30 %, and which on the inner surface of the composite tube comprises a plurality of depressions oriented towards the outer wall of the composite tube.

2. The composite tube according to claim 1, wherein the thermal shock resistance according to DIN EN 993-11 of the composite tube is greater than 50 K/h.

3. The composite tube according to at least one of claims 1 to 2, wherein the depressions have a depth of 0.5 mm to 2 mm.

4. The composite tube according to at least one of claims 1 to 3, wherein the depressions are uniformly distributed over the inner surface of the composite tube.

5. The composite tube according to at least one of claims 1 to 4, wherein the depressions are nonuniformly distributed over the inner surface of the composite tube.

6. The composite tube according to at least one of claims 1 to 5, wherein the inner surface of the composite tube is provided with depressions to an extent of 10% to 95% based on the total inner surface of the composite tube.

7. The composite tube according to at least one of claims 1 to 6, wherein the depressions are concave.

8. The composite tube according to at least one of claims 1 to 7, wherein the depressions have a construction that is circular in cross section and have a diameter of 2 mm to 30 mm.

9. The composite tube according to at least one of claims 1 to 8, wherein the total wall thickness of the composite tube is 0.5 mm to 50 mm.

10. The composite tube according to at least one of claims 1 to 9, wherein the tube internal diameter of the composite tube is 10 mm to 1000 mm.

11. The composite tube according to at least one of claims 1 to 10, wherein the employed oxidic fiber composite ceramic is $SiC/Al_2O_3$, $SiC/mullite$, $C/Al_2O_3$, $C/mullite$, $Al_2O_3/Al_2O_3$, $Al_2O_3/mullite$, $mullite/Al_2O_3$ and/or mullite/mullite.

12. The composite tube according to at least one of claims 1 to 11, wherein the composite tube contains two layers, namely an inner layer and an outer layer, wherein the inner layer is constructed from nonporous monolithic oxide ceramic and the outer layer is constructed from oxidic fiber composite ceramic.

13. The composite tube according to at least one of claims 1 to 11, wherein the composite tube has a structure in which the nonporous monolithic oxide ceramic is covered by oxidic fiber composite ceramic.

14. The composite tube according to at least one of claims 1 to 13, wherein the inner layer has a minimum layer thickness of 0.5 mm to 45 mm.

15. The use of the composite tube according to at least one of claims 1 to 14 in the production of synthesis gas by reforming of hydrocarbons with steam and/or carbon dioxide, coproduction of hydrogen and pyrolysis carbon by pyrolysis of hydrocarbons, production of hydrocyanic acid from methane and ammonia or from propane and ammonia, production of olefins by steamcracking of hydrocarbons and/or coupling of methane to ethylene, acetylene and to benzene.

16. The use of the composite tube according to at least one of claims 1 to 14 as reaction tubes in reactors with axial temperature control, countercurrent reactors, membrane reactors, jet tubes, flame tubes and/or rotary tubes for rotary tube furnaces.

17. A process for producing the multilayered composite tube according to claim 1, wherein the depressions are impressed by pressing processes.

**Revendications**

1. Tube composite multicouche ayant un coefficient de transmission de la chaleur, selon la détermination, indiquée dans la description, du coefficient de transmission de la chaleur, > 500 $W/m^2/K$, contenant au moins deux couches, qui dans sa structure comprend, au-dessus de la section transversale de la paroi du tube composite servant de couche intérieure, une céramique d'oxyde monolithique poreuse non ouverte, qui est entourée d'une couche extérieure constituée d'une céramique de type oxyde renforcée par des fibres, cette couche extérieure présentant une porosité ouverte $\varepsilon$ selon DIN V ENV 1389 de 5 % < $\varepsilon$ < 50 %, de préférence 10 % < $\varepsilon$ < 30 %, qui sur la surface intérieure du tube composite comprend plusieurs renfoncements dirigés vers la paroi extérieure du tube composite.

2. Tube composite selon la revendication 1, **caractérisé en ce que** la résistance au choc thermique dans le sens de DIN EN 993-11 du tube composite est supérieure à 50 K/h.

3. Tube composite selon au moins l'une des revendications 1 à 2, **caractérisé en ce que** les renfoncements présentent une profondeur de 0,5 mm à 2 mm.

4. Tube composite selon au moins l'une des revendications 1 à 3, **caractérisé en ce que** les renfoncements sont uniformément répartis sur la surface intérieure du tube composite.

5. Tube composite selon au moins l'une des revendications 1 à 4, **caractérisé en ce que** les renforcements sont répartis d'une manière non uniforme sur la surface intérieure du tube composite.

6. Tube composite selon au moins l'une des revendications 1 à 5, **caractérisé en ce que** la surface intérieure du tube composite est pourvue de renforcements sur 10 % à 95 % de la surface intérieure totale du tube composite.

7. Tube composite selon au moins l'une des revendications 1 à 6, **caractérisé en ce que** les renforcements ont une forme concaves.

8. Tube composite selon au moins l'une des revendications 1 à 7, **caractérisé en ce que** les renforcements ont une section transversale circulaire et présentent un diamètre de 2 mm à 30 mm.

9. Tube composite selon au moins l'une des revendications 1 à 8, **caractérisé en ce que** l'épaisseur de paroi totale du tube composite est de 0,5 mm à 50 mm.

10. Tube composite selon au moins l'une des revendications 1 à 9, **caractérisé en ce que** le diamètre intérieur du tube composite est de 10 mm à 1 000 mm.

11. Tube composite selon au moins l'une des revendications 1 à 10, **caractérisé en ce qu'**on utilise en tant que céramique de type oxyde renforcée par des fibres $SiC/Al_2O_3$, $SiC/mullite$, $C/Al_2O_3$, $C/mullite$, $Al_2O_3/Al_2O_3$, $Al_2O_3/mullite$, $mullite/Al_2O_3$ et/ou mullite/mullite.

12. Tube composite selon au moins l'une des revendications 1 à 11, **caractérisé en ce que** le tube composite contient deux couches, une couche intérieure et une couche extérieure, la couche intérieure étant constituée d'une céramique d'oxyde monolithique non poreuse et la couche extérieure étant constituée d'une céramique de type oxyde renforcée par des fibres.

13. Tube composite selon au moins l'une des revendications 1 à 11, **caractérisé en ce que** le tube composite présente une structure dans laquelle la céramique d'oxyde monolithique non poreuse est recouverte d'une céramique de type oxyde renforcée par des fibres.

14. Tube composite selon au moins l'une des revendications 1 à 13, **caractérisé en ce que** la couche intérieure présente une épaisseur minimale de couche de 0,5 mm à 45 mm.

15. Utilisation du tube composite selon au moins l'une des revendications 1 à 14 pour la fabrication de gaz de synthèse par reformage d'hydrocarbures avec de la vapeur d'eau et/ou du dioxyde de carbone, pour la production jumelée d'hydrogène et de carbone de pyrolyse par la pyrolyse d'hydrocarbures, pour la fabrication d'acide cyanhydrique à partir de méthane et d'ammoniac ou de propane et d'ammoniac, pour la fabrication d'oléfines par vapocraquage d'hydrocarbures et/ou pour le couplage de méthane en éthylène, acétylène et benzène.

16. Utilisation du tube composite selon au moins l'une des revendications 1 à 14 en tant que tubes de réaction dans des réacteurs à régulation axiale de la température, dans des réacteurs à contre-courant, dans des réacteurs à membranes, dans des lances de pulvérisation, dans des carneaux et/ou des tubes pour fours tubulaires rotatifs.

17. Procédé de fabrication du tube composite multicouche selon la revendication 1, **caractérisé en ce qu'**on estampe les renforcements par un procédé de pressage.

Abbildung 1

Abbildung 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2016184776 A1
- WO 2015052066 A1
- WO 2017007649 A1
- WO 2017178551 A1
- WO 2017007649 A

**In der Beschreibung aufgeführte Nicht-Patentliteratur**

- Risswiderstand keramischer Faserverbundwerkstoffe. **M. KUNTZ.** Dissertation. Universität Karlsruhe, Shaker Verlag, 1996
- Grundlagen; Verbindungselemente; Gehäuse, Behälter, Rohrleitungen und Absperrvorrichtungen. **W. TOCHTERMANN ; F. BODENSTEIN.** Konstruktionselemente des Maschinenbaues. Springer-Verlag, 1979
- **DARKEN, L. S. ; GURRY, R. W.** Physica/ chemistry of metals. McGraw-Hill, 1953
- Wärmedurchgang. VDI-Wärmeatlas. 1997
- Informationszentrum Technische Keramik (IZTK): Brevier technische Keramik. Fahner Verlag, 2003
- **SCHMÜCKER, M.** Faserverstärkte oxidkeramische Werkstoffe. *Materialwissenschaft und Werkstofftechnik,* 2007, vol. 38 (9), 698-704